Europäisches Patentamt

European Patent Office

Office européen des brevets

(19)

(11) **EP 0 682 937 B1**

(12) **FASCICULE DE BREVET EUROPEEN**

(45) Date de publication et mention
de la délivrance du brevet:
**02.12.1998 Bulletin 1998/49**

(51) Int. Cl.⁶: **A61K 7/135**

(21) Numéro de dépôt: **95401033.6**

(22) Date de dépôt: **04.05.1995**

(54) **Procédé pour améliorer les résultats des traitements cosmétiques effectués sur cheveux décolerés,grace à la décolaration par irradiation avec un faisceau laser**

Verfahren zur Verbesserung der kosmetischen behandlungen von entfärbten Haaren, dank einer Haarenentfärbung durch Laserausstrahlung.

Process for improving the results of cosmetic treatments on bleached hair,thanksto bleaching by laser irradiation.

(84) Etats contractants désignés:
**AT BE CH DE ES FR GB IT LI NL SE**

(30) Priorité: **04.05.1994 FR 9405470**
**04.05.1994 FR 9405469**
**04.05.1994 FR 9405468**

(43) Date de publication de la demande:
**22.11.1995 Bulletin 1995/47**

(73) Titulaire: **L'OREAL, S.A.**
**75008 Paris (FR)**

(72) Inventeurs:
• **Caisey, Laurence**
**F-94400 Vitry-sur-Seine (FR)**

• **Monnais, Christian**
**F-92200 Neuilly-sur-Seine (FR)**
• **Samain, Henri**
**F-91570 Bièvres (FR)**

(74) Mandataire:
**Tonnellier, Jean-Claude**
**Nony & Associés,**
**29, rue Cambacérès**
**75008 Paris (FR)**

(56) Documents cités:
**WO-A-91/06279**    **WO-A-94/10874**
**DE-A- 3 330 293**    **US-A- 4 792 341**

## Description

L'invention concerne un procédé pour améliorer les résultats des traitements cosmétiques effectués sur cheveux décolorés. On a en effet découvert que les résultats des traitements cosmétiques effectués sur cheveux décolorés étaient nettement améliorés lorsque l'on effectue ledit traitement sur des cheveux décolorés par irradiation avec un faisceau laser.

On sait que pour décolorer ou éclaircir les cheveux, on utilise classiquement un traitement chimique à l'aide d'un agent oxydant, tel que le peroxyde d'hydrogène ou des persels, qui détruit au moins une partie des produits colorants naturels et/ou artificiels présents dans les cheveux.

La méthode classique de décoloration chimique des cheveux nécessite l'emploi d'agents oxydants relativement puissants et/ou concentrés, qui ont pour effet de dégrader non seulement les substances colorantes, mais aussi la fibre kératinique des cheveux. Il en résulte que les cheveux ainsi décolorés sont fragiles et doivent ensuite être traités avec précaution.

Pour ces raisons, il est difficile ou parfois impossible d'obtenir de bons résultats avec les traitements cosmétiques appliqués sur cheveux décolorés par voie chimique.

Par exemple, il est impossible d'appliquer à de tels cheveux les traitements de déformation permanente utilisés pour les cheveux naturels, car ces traitements consistent à appliquer sur les cheveux des agents réducteurs, à des pH relativement élevés, puis un agent oxydant, qui ont des effets nettement agressifs sur la fibre kératinique des cheveux. En fait, l'application d'un traitement classique de déformation permanente sur une chevelure décolorée par voie chimique provoque des dégradations irréversibles et même des cassures des cheveux.

Cela explique que les coiffeurs expérimentés n'acceptent pas d'effectuer une opération de déformation permanente immédiatement ou peu de temps après une décoloration par voie chimique. Pour effectuer une permanente sur des cheveux ainsi fragilisés, ils utilisent des compositions pour permanentes "faibles", c'est-à-dire nettement moins dosées en agent réducteur et/ou présentant un pH voisin de la neutralité. Dans ces conditions, les dégradations des cheveux sont plus limitées. Toutefois, les cheveux ainsi traités présentent une frisure en zigzag peu esthétique. Les boucles obtenues n'ont pas l'élasticité de celles obtenues par déformation permanente des cheveux naturels. En outre, lorsque les cheveux ont été fortement décolorés, il est possible que la permanente n'induise aucune frisure. On rappelle ici que l'opération de déformation permanente des cheveux est couramment appelée "permanente", et que les cheveux ayant subi une telle opération sont dits "permanentés".

Par ailleurs, on sait qu'une proportion importante de personnes souhaite obtenir une chevelure dite "méchée", c'est-à-dire une chevelure qui n'est pas totalement décolorée, mais dans laquelle seulement certaines mèches de cheveux sont décolorées. Il s'agit d'une opération dite de "méchage". Le cas des chevelures méchées est particulièrement délicat, car on retrouve alors sur une même chevelure des cheveux naturels et des cheveux très décolorés. Les cheveux naturels ont besoin, pour friser, d'un produit de permanente "fort", tandis que pour limiter les problèmes de dégradation, il conviendrait de n'appliquer sur les cheveux décolorés qu'un produit de permanente "faible". Une telle procédure n'étant pas utilisable en pratique, on résout généralement ces difficultés en enduisant les mèches décolorées à l'aide d'une substance protectrice, puis on applique une composition de permanente assez forte sur l'ensemble de la chevelure. Avec cette technique, les cheveux naturels sont assez bien permanentés, mais les mèches décolorées sont en général assez abîmées et présentent des boucles peu esthétiques. En outre, cette technique prend du temps car elle nécessite d'enduire avec le produit protecteur chaque mèche décolorée. Le résultat final est assez peu satisfaisant car certaines mèches (les mèches naturelles) frisent bien tandis que d'autres mèches (les mèches décolorées) frisent peu.

En fait, dans le cas de pratiquement tous les traitements cosmétiques appliqués aux cheveux décolorés, on observe des difficultés de réalisation ou des résultats décevants.

On sait par exemple que certaines personnes souhaitent obtenir non pas des mèches décolorées mais des mèches colorées ayant une nuance différence de celle du reste de la chevelure. Pour cela, il convient d'abord de décolorer lesdites mèches, puis d'appliquer sur la chevelure un agent colorant. On constate que sur les mèches décolorées, la teinte n'est pas stable. Par ailleurs, avec les colorants d'oxydation qui ne développent une coloration qu'en présence d'un agent oxydant, les mèches préalablement décolorées sont davantage abimées car elles sont plus sensibles à l'effet inévitablement dégradant des traitements par colorants d'oxydation. En outre, certains colorants colorent difficilement les cheveux préalablement décolorés. De plus, les mèches décolorées puis teintes présentent des problèmes de tenue de colorant. En effet, par lavage à l'aide d'un shampooing, elles ont tendance à se décolorer rapidement.

L'application d'agents de conditionnement des cheveux sur cheveux décolorés conduit également à des résultats décevants, car ces cheveux sont facilement emmêlés et l'application d'agents de conditionnement, tels que des démêlants ou des mousses à rincer, n'empêchent pas la cassure des cheveux décolorés, lors du démêlage, en raison de leur fragilité.

De même, après l'application de certaines laques pour cheveux, les cheveux décolorés liés à des cheveux non décolorés par des grains de laque peuvent se trouver arrachés lors du peignage.

Des problèmes analogues peuvent être rencontrés après application de lotions de mises en plis. En outre, les cheveux décolorés étant plus mous que les cheveux naturels, il en résulte que la coiffure n'a pas un aspect homogène, même après application d'une laque ou d'une lotion de mise en plis, en particulier lorsque la chevelure est méchée.

Par ailleurs, l'application de shampooings qui contiennent des détergents, est moins bien supportée par les fibres kératiniques des cheveux décolorés, en particulier dans le cas de shampooings fréquents. On voit donc que pratiquement tous les traitements cosmétiques appliqués à des cheveux décolorés posent des problèmes difficiles ou parfois même impossibles à résoudre.

L'invention permet de remédier à ces divers inconvénients grâce à la découverte qu'il est possible de décolorer les cheveux par irradiation avec un rayonnement laser, et cela sans altérer les propriétés mécaniques et physico-chimiques des cheveux.

L'invention permet donc d'appliquer, avec de bons résultats, et sans prendre de précautions particulières, les divers traitements cosmétiques usuels, y compris ceux qui viennent d'être mentionnés. On obtient ces résultats améliorés en appliquant lesdits traitements à des cheveux décolorés non pas par voie chimique mais par irradiation à l'aide d'un faisceau laser, dans des conditions particulières qui permettent d'obtenir, sur tous types de cheveux, colorés naturellement ou artificiellement, une décoloration d'intensité facilement contrôlable, sans dégradation notable de la fibre kératinique, et avec une durée de traitement raisonnable. Ce procédé de décoloration est particulièrement bien adapté à l'obtention d'une chevelure méchée. En outre, les cheveux ainsi décolorés, ayant conservé les propriétés mécaniques et physico-chimiques qu'ils avaient avant la décoloration, peuvent être soumis sans délai à d'autres traitements cosmétiques, sans qu'il soit nécessaire d'utiliser des substances protectrices, et l'on obtient des résultats améliorés pour l'ensemble des traitements cosmétiques usuels.

La possibilité théorique de décolorer des cheveux avec un rayonnement laser a été mentionnée dans la publication Tech. News, *Laser Focus*, Vol.19, No. 9, p.26, Septembre 1983. En outre, dans le brevet US-4 792 341, on a décrit un dispositif expérimental permettant d'étudier la destruction de la mélanine des cheveux à l'aide d'un rayonnement laser. En fait, ce brevet US ne décrit ni un procédé ni un dispositif permettant, en pratique, l'application de l'irradiation laser à la décoloration des cheveux.

On a maintenant découvert qu'il est possible de décolorer des mèches de cheveux, quelle que soit leur couleur d'origine (naturelle ou artificielle), sans dégradation de la fibre kératinique, en soumettant successivement des parties des mèches de cheveux à traiter à une irradiation par un faisceau laser à condition de choisir une puissance du rayonnement laser adaptée au type de cheveux à décolorer.

Des études sur cheveux isolés de diverses origines (cheveux naturels européens, japonais, mexicains et scandinaves) ont permis d'étudier la puissance lumineuse nécessaire pour obtenir une bonne décoloration des cheveux sans dégradation de la fibre kératinique, avec une seule impulsion laser (tir monocoup). On a constaté que les cheveux très foncés (japonais et mexicains) ne se décolorent pas suffisamment en profondeur ou éclatent sous l'irradiation lorsqu'on augmente encore la puissance par unité de surface, pour une durée d'impulsion donnée. En utilisant une puissance crête inférieure à la puissance crête la plus élevée qui, en tir monocoup, pour la durée d'impulsion utilisée et pour le type de cheveux étudié, ne provoque pas l'éclatement de la fibre kératinique, on a ensuite effectué des tirs successifs, en séquences, sur une même zone des cheveux isolés traités. On a découvert que les cheveux isolés, même les cheveux très foncés du type japonais ou mexicain, pouvaient être décolorés sans dommage en abaissant ainsi la puissance crête et en effectuant plusieurs passages successifs sur une zone traitée, ce qui permet de décolorer d'abord les couches superficielles, puis les couches plus profondes et finalement les cheveux peuvent être décolorés totalement.

Des études analogues faites sur des mèches de cheveux ont permis de constater qu'il est possible de décolorer les cheveux disposés en mèches, à condition de sélectionner une puissance du rayonnement laser adaptée au type de cheveux traités. Cette puissance doit être suffisante pour dégrader ou détruire la mélanine, mais ne doit pas dépasser un certain seuil. On a découvert que plus la couleur naturelle des cheveux est foncée, plus ce seuil doit être faible. On arrive donc à ce résultat surprenant que plus les cheveux sont difficiles à décolorer, plus la puissance du rayonnement laser doit être modérée.

Comme cela apparaîtra évident pour les spécialistes, c'est en fait l'énergie fournie aux grains de mélanine, sur une durée de temps suffisamment courte, qui doit être suffisante pour dégrader ou détruire la mélanine. C'est donc en réalité la densité d'énergie fournie par unité de surface, en un temps suffisamment court, qui doit atteindre un seuil suffisamment élevé pour que les cheveux puissent être décolorés. Dans la présente demande, lorsqu'on parle de "puissance" ou de "puissance crête", il faut comprendre qu'il s'agit d'une simplification de langage, car c'est en fait l'énergie fournie lors de chaque impulsion qui est importante, et il faut donc tenir compte de la durée de l'impulsion, qui doit être toutefois non supérieure à une microseconde environ (durée approximative du temps de relaxation de la mélanine) dans le cas de la décoloration des cheveux naturels.

On a également découvert qu'il est possible de décolorer de façon analogue les cheveux colorés artificiellement, à condition d'opérer, comme pour les cheveux non teints, en adaptant la puissance du rayonnement laser à la couleur naturelle des cheveux. Ce n'est qu'après avoir effectué cette étape préliminaire, correspondant à la dégradation de la mélanine, que l'on peut entreprendre la décoloration proprement dite, correspondant à la dégradation du colorant artificiel. En effet, la dégradation des colorants artificiels nécessite des énergies plis importantes que la dégradation de la mélanine, de sorte que si l'on cherchait à dégrader directement lesdits colorants, les cheveux seraient détruits par éclatement de la fibre kératinique, comme dans les expériences rapportées ci-dessus.

On sait par ailleurs que certaines personnes âgées ont une chevelure "blanche" qui a en fait une teinte jaunâtre peu esthétique. Le procédé de décoloration par irradiation laser permet de transformer cette coloration jaunâtre en blanc pur.

L'invention a donc pour objet un procédé de traitement des cheveux comprenant une décoloration desdits cheveux pour obtenir des cheveux décolorés, et l'application d'un traitement cosmétique, autre qu'une décoloration, sur lesdits cheveux décolorés, caractérisé par le fait que, dans le but d'améliorer les résultats dudit traitement cosmétique, ladite décoloration consiste essentiellement à irradier lesdits cheveux à l'aide d'un rayonnement laser.

L'invention a en particulier pour objet un procédé tel que défini ci-dessus dans lequel les cheveux auxquels le traitement est appliqué ont été décolorés par un procédé dans lequel on décolore au moins partiellement au moins une mèche ou partie de mèche de cheveux par irradiation de ladite mèche ou partie de mèche à l'aide d'un faisceau laser de puissance suffisante pour décolorer les cheveux, ce procédé de décoloration comportant les étapes suivantes :

- on traite une zone de ladite partie de mèche par irradiation à l'aide d'un faisceau laser émis sous forme d'impulsions pour décolorer au moins partiellement les cheveux de ladite zone, par dégradation de la mélanine des cheveux,
- le cas échéant, par déplacement relatif de ladite mèche par rapport audit faisceau laser, on traite successivement, de façon analogue, une ou plusieurs autres zones de façon à traiter la totalité de ladite partie de mèche,
- on répète éventuellement les traitements précédents jusqu'à obtention du degré de décoloration souhaité pour ladite mèche ou partie de mèche,
- on opère avec un rayonnement laser de puissance suffisante pour fournir, par impulsion, une densité d'énergie de 0,1 à 1,2 J/cm$^2$ à 532 nm, ladite densité d'énergie choisie n'étant pas supérieure à un seuil au-delà duquel la fibre kératinique des cheveux est endommagée, ledit seuil étant d'autant plus faible que la couleur naturelle des cheveux à traiter est plus foncée.

On sait qu'un laser se compose essentiellement d'un milieu actif rendu amplificateur par un mécanisme de pompage fournissant de l'énergie aux atomes de façon sélective, ledit milieu actif étant enfermé dans une cavité résonnante. Le milieu actif est alors capable d'émettre un faisceau lumineux, sensiblement monochromatique, polarisé et cohérent. En raison de cette cohérence, un faisceau laser concentre une énergie nettement plus importante que celle d'un rayonnement émis par une source lumineuse classique.

Certains lasers, notamment ceux à milieu actif solide, sont capables d'émettre un rayonnement laser sous la forme d'impulsions très brèves (généralement entre la femtoseconde et la microseconde). La concentration de l'énergie sur des intervalles de temps aussi brefs confère à l'impulsion laser une puissance, dite puissance crête, considérable. On utilise de préférence dans le procédé de l'invention des lasers permettant la production d'impulsions contrôlées. On peut utiliser par exemple des lasers à rubis ou des lasers dont le milieu actif contient des ions de terres rares ou d'actinides, par exemple un laser à néodyme. La réalisation de tels lasers est bien connue. Les ions actifs peuvent être insérés dans une matrice cristalline telle que le grenat d'yttrium-aluminium (en abrégé YAG), ou dans une matrice amorphe telle qu'un verre. On règle la fréquence de récurrence des impulsions à l'aide d'une lampe de pompage à éclairs. On peut régler l'énergie disponible à l'aide de systèmes classiques, notamment des polariseurs.

On utilise de préférence des lasers émettant dans l'ultraviolet proche, dans le visible ou dans le proche infrarouge, par exemple à des longueurs d'onde de 300 à 1100 nm. On peut utiliser par exemple un laser YAG-néodyme qui émet à 1,06 µm éventuellement avec un multiplicateur de fréquence qui permet par exemple d'obtenir une émission de longueur d'ondes 532 nm (fréquence double) ou de 355 nm (fréquence triple).

La détermination du couple puissance crête-durée d'impulsion (par exemple la puissance crête du faisceau laser pour une durée d'impulsion donnée) peut être aisément déterminée en tenant compte des conditions concernant la densité d'énergie par impulsion, en fonction de la couleur naturelle des cheveux, même s'il s'agit de cheveux teints. Plus précisément, il est souhaitable de ne pas dépasser une densité d'énergie maximum par impulsion qui est de l'ordre de :

- 0,35 J/cm$^2$ pour les cheveux brun foncé (de type japonais ou mexicain),
- 0,4 J/cm$^2$ pour les cheveux châtain foncé,
- 0,5 J/cm$^2$ pour les cheveux châtain clair,
- 0,7 J/cm$^2$ pour les cheveux blond foncé,
- 1,2 J/cm$^2$ pour les cheveux blond clair.

Les diverses données fournies ci-dessus concernant la densité d'énergie ont été établies pour un rayonnement de longueur d'onde 532 nm. Lorsque la longueur d'onde utilisée est différente, il convient d'appliquer un facteur de correction

$$\frac{\lambda \text{ nm}}{532}$$

comme cela est exposé plus en détail dans la partie expérimentale ci-après.

Par ailleurs, on rappelle que les différentes teintes de cheveux peuvent être définies de façon objective par la luminance (L), selon le système de coordonnées colorimétriques (L,a,b) du C.I.E. (Comité International de l'Eclairage). Dans la présente demande, les teintes des cheveux mentionnées correspondent aux gammes de luminance mentionnées ci-après :

| Type de cheveux | L |
|---|---|
| japonais ou mexicain | inférieure à 18 |
| châtain foncé | 18-20 |
| châtain clair | 22-24 |
| blond foncé | 28-35 |
| blond clair | 45-52 |

Le procédé décrit ci-dessus, qui correspond à une décoloration au moins partielle par dégradation de la mélanine des cheveux, doit être effectué dans tous les cas, même si l'on traite les cheveux teints à l'aide d'un agent colorant, et dans ce dernier cas, le procédé de décoloration comporte une étape supplémentaire d'irradiation des cheveux, analogue à celle décrite ci-dessus, mais par un rayonnement laser fournissant une densité d'énergie plus élevée, suffisante pour détruire ou dégrader l'agent colorant ; cette densité d'énergie est notamment au moins égale à 0,8 J/cm$^2$. Elle est généralement inférieure à 2 J/cm$^2$ à 532 nm.

Pour mettre en oeuvre l'étape préliminaire de dégradation de la mélanine, il suffit de connaître la couleur naturelle des cheveux teints à traiter, et on applique alors des conditions d'irradiation convenant pour les cheveux ayant cette couleur naturelle, lesdites conditions ayant été déterminées préalablement, une fois pour toutes, par de simples expériences de routine.

La durée des impulsions peut aller par exemple de 10 picosecondes à 100 nanosecondes.

Le procédé de décoloration par laser nécessite évidemment de décolorer la chevelure par mèches, en irradiant successivement des zones de ladite mèche.

Généralement, la surface d'irradiation peut varier dans la gamme de 0,1-2cm$^2$.

Ce procédé est donc particulièrement bien adapté à l'obtention de chevelures méchées. Pour traiter successivement les zones à décolorer par déplacement relatif d'une mèche par rapport au faisceau laser, on peut déplacer la mèche par rapport à l'appareil utilisé pour le traitement, ou vice-versa, et/ou faire varier périodiquement la direction du faisceau laser de façon à effectuer des balayages successifs de la zone traitée. Cette variation périodique de la direction du faisceau laser peut être obtenue par exemple à l'aide d'un miroir oscillant.

On a constaté que lorsqu'on effectue la décoloration par des tirs laser en séquence, les cheveux avaient tendance à s'échauffer localement. Pour que cet échauffement local ne soit pas dommageable pour les cheveux, il convient de limiter la fréquence de récurrence des impulsions, et/ou de procéder à des déplacements relatifs suffisants de la mèche de cheveux traitée par rapport au faisceau laser de façon à éviter des échauffements cumulés, après quoi on peut revenir sur une zone précédemment traitée, ayant eu le temps de refroidir suffisamment, pour compléter le traitement, et ainsi de suite. Une autre solution pour éviter l'échauffement local des cheveux est de refroidir les cheveux dans la zone traitée. On peut refroidir les cheveux dans la zone traitée notamment par circulation d'un fluide. La solution la plus simple est de créer un flux gazeux baignant les cheveux dans la zone traitée, par exemple par aspiration de l'air ou encore en soufflant un gaz, éventuellement refroidi au préalable, tel que de l'air, de l'azote, de l'hélium, du dioxyde de carbone, etc. On peut bien entendu utiliser un gaz chargé de vapeur d'eau ou chargé de particules liquides (aérosol), par exemple des gouttelettes d'eau.

En pratique, il est facile de déterminer une fréquence convenable de récurrence des impulsions et/ou une vitesse appropriée de déplacement de la mèche de cheveux par rapport au faisceau laser, par de simples expériences de routine, ladite fréquence convenable et/ou ladite vitesse appropriée étant celles pour lesquelles on n'observe pas d'échauffement dommageable des cheveux. On peut choisir par exemple une fréquence de récurrence des impulsions pouvant aller de 5 à 50 Hz. La méthode consistant à faire varier périodiquement la direction du faisceau laser, comme indiqué ci-dessus, constitue également l'un des moyens de lutter contre l'échauffement local des cheveux. Dans le cas où l'on applique des mesures de refroidissement des cheveux, il est possible d'opérer avec des fréquences de récurrence des impulsions supérieures à celles qui viennent d'être indiquées. La fréquence des impulsions, dans ce cas, peut aller par exemple jusqu'à 1000 Hz, notamment de 10 à 100 Hz environ. La capacité du système de refroidissement à évacuer la chaleur produite, de façon à éviter la dégradation thermique des cheveux, doit évidemment être adaptée au choix de la fréquence des impulsions, qui elle-même, conditionne la durée du traitement.

Les cheveux traités peuvent être secs ou humides.

Pour pouvoir décolorer la mèche de cheveux traitée dans de bonnes conditions, il est préférable de disposer cette mèche sous la forme d'une nappe de cheveux, et on peut alors décolorer les cheveux de la zone traitée en irradiant au moins une des faces de ladite nappe. De préférence, l'épaisseur de la nappe de cheveux correspond à l'épaisseur de 3 à 20 cheveux superposés environ.

Selon un mode de réalisation particulier, l'étape de décoloration du procédé de décoloration laser est caractérisée par le fait

- que l'on opère à l'aide d'un dispositif de traitement comprenant un réceptacle pour la mèche de cheveux à traiter, la surface dudit réceptacle comportant un orifice pour la sortie dudit faisceau laser,
- que l'on applique, sur au moins une partie de leur longueur, les cheveux de ladite mèche étalés en nappe sur la surface dudit réceptacle, de façon qu'une zone à traiter soit en regard dudit orifice,
- que l'on procède à l'irradiation de ladite zone,
- et que par déplacements relatifs de ladite mèche par rapport au faisceau laser, on procède successivement à l'irradiation des autres zones à traiter. On décrira ci-après des dispositifs permettant une telle mise en oeuvre. Avec ces dispositifs, les déplacements relatifs de la mèche par rapport au faisceau laser peuvent être obtenus par des déplacements relatifs de la mèche par rapport audit réceptacle et/ou par variations périodiques de la direction du faisceau laser, comme indiqué précédemment.

L'un des avantages de la décoloration des cheveux par irradiation avec un faisceau laser est qu'il est possible de contrôler en continu la décoloration produite et d'arrêter le traitement au degré de décoloration choisi.

Comme indiqué ci-dessus, l'avantage principal de la décoloration par irradiation laser est que les cheveux ne sont pas dégradés et conservent les propriétés mécaniques et physico-chimiques qu'ils avaient avant traitement. Il s'agit là d'un avantage considérable puisque l'on peut effectuer sur les cheveux ainsi décolorés d'autres traitements ayant un effet dégradant, sans observer nécessairement un délai minimum entre les deux opérations et sans prendre de précautions particulières. C'est ainsi qu'il est possible de permanenter les cheveux décolorés par irradiation avec un faisceau laser soit immédiatement soit peu de temps après la décoloration. Il n'est pas nécessaire de prendre des mesures particulières de protection des mèches décolorées avant d'appliquer la composition de permanente. Il est possible, et même souhaitable, d'utiliser des compositions de permanente à dosage normal, pour cheveux naturels, ce qui permet d'obtenir de bons résultats aussi bien pour les mèches décolorées que pour le restant de la chevelure.

L'invention permet donc notamment de mettre en oeuvre un procédé pour permanenter des cheveux décolorés, caractérisé par le fait que l'on effectue le traitement de déformation permanente des cheveux, selon les méthodes connues, sur des cheveux décolorés par irradiation avec un faisceau laser.

Pour mettre en oeuvre l'étape de décoloration des cheveux définie précédemment, on peut utiliser un dispositif qui comprend

- un corps muni d'un réceptacle, ledit réceptacle étant destiné à servir de logement, sur au moins une partie de la longueur des cheveux, pour une mèche de cheveux à traiter étalée en nappe,
- des moyens destinés à acheminer un faisceau laser de façon à irradier au moins une zone d'une mèche de cheveux disposée dans ledit réceptacle,
- et, le cas échéant, des moyens pour refroidir lesdits cheveux dans la zone ou les zones d'irradiation.

Dans des modes de réalisation particuliers, ce dispositif peut encore présenter les caractéristiques suivantes, prises isolément ou, le cas échéant, en combinaison :

- ledit réceptacle comprend une rainure à fond large, permettant d'étaler ladite nappe sur ledit fond ;
- ladite rainure peut être associée à une pièce dégageable de forme coopérante capable d'insertion dans ladite rainure en ménageant, entre ladite pièce et le fond de la rainure, un espace constituant un logement pour ladite nappe ; ladite pièce est par exemple dégageable par actionnement d'un levier permettant d'introduire la mèche à traiter dans le réceptacle ou de l'en retirer ; le fond de ladite rainure et/ou une face de ladite pièce en regard dudit fond peut comporter un orifice pour la sortie d'un faisceau laser vers la zone à irradier ; de même, le fond de ladite rai-

EP 0 682 937 B1

nure ou une face de ladite pièce en regard dudit fond peut comporter un orifice pour la création d'un flux gazeux (par aspiration ou par soufflage) ;

- dans un autre mode de réalisation, ledit réceptacle comprend une rainure en forme de fente fine débouchant à la surface dudit corps et ayant une profondeur suffisante pour permettre l'insertion de ladite nappe sur la totalité de sa largeur ; de façon analogue à celle mentionnée ci-dessus pour le premier mode de réalisation, l'une au moins des faces en regard de ladite fente fine peut comporter un orifice pour la sortie d'un faisceau laser ; et l'une au moins des faces en regard de ladite fente fine peut comporter un orifice pour la création d'un flux gazeux.

Il faut noter enfin que le canal laser et le conduit de création d'un flux gazeux peuvent être confondus.

Bien entendu, le réceptacle doit constituer un espace confiné étanche à la lumière pour éviter que le rayonnement laser puisse provoquer des dommages à l'utilisateur ou à sa clientèle.

La présente invention a également pour objet l'utilisation de la décoloration des cheveux à l'aide d'un rayonnement laser pour améliorer les procédés de traitements cosmétiques effectués sur cheveux décolorés.

Par le procédé qui vient d'être décrit en détail, il est possible d'effectuer sur les cheveux ainsi décolorés et dans de bonnes conditions divers traitements cosmétiques nécessaires aux soins de la chevelure. Ces traitements sont notamment la réalisation d'une déformation permanente, d'une coloration, d'un shampooing, ou encore l'application d'une laque, d'une composition de conditionnement, d'une lotion de mise en plis, etc.

Les procédés techniques de traitements cosmétiques dont il est possible d'améliorer les résultats grâce au procédé de l'invention sont connus en soi. On rappelle ci-après à titre d'illustration le cas de la déformation permanente des cheveux.

L'étape de déformation permanente des cheveux qui ont été décolorés par le procédé indiqué ci-dessus peut être effectuée selon les méthodes usuelles, ce qui n'est pas possible, comme rappelé ci-dessus, lorsque les cheveux ont été décolorés par voie chimique. On peut effectuer l'opération de déformation permanente immédiatement après la décoloration ou quelques jours ou quelques semaines plus tard.

On rappelle que la déformation permanente des cheveux est réalisée de façon usuelle selon un procédé en deux étapes qui consiste, dans un premier temps, à traiter les cheveux avec un agent réducteur, pour réaliser l'ouverture des liaisons disulfure de la kératine des cheveux, puis après avoir de préférence rincé la chevelure, à appliquer sur les cheveux, soumis à une tension mécanique, une composition oxydante (étape dite de fixation) de façon à reconstituer lesdites liaisons disulfures et à donner ainsi aux cheveux la forme désirée. Cette technique permet de réaliser aussi bien l'ondulation des cheveux que leur défrisage ou leur décrêpage.

Les compositions réductrices et oxydantes utilisables dans les procédés de déformation permanente des cheveux sont bien connues et décrites notamment dans les ouvrages de cosmétologie. L'agent réducteur est par exemple l'acide thioglycolique, l'acide thiolactique, un sulfite ou bisulfite alcalin ou d'ammonium.

La mise sous tension des cheveux est effectuée par exemple, de façon connue, en les enroulant sur des bigoudis. Dans le cas du défrisage, la mise sous tension des cheveux est obtenue par exemple par lissage des cheveux avec un peigne.

L'agent oxydant est notamment le peroxyde d'hydrogène ou un persel.

On va maintenant illustrer l'invention en faisant référence aux dessins annexés dans lesquels :

- la figure 1 est une vue schématique d'un premier mode de réalisation du dispositif de l'invention, qui comprend à son extrémité un réceptacle sous forme de rainure à fond large et une pièce coopérante actionnable par un levier,
- la figure 2 est une vue partielle de dessus de l'extrémité, portant le réceptacle, du dispositif de la figure 1,
- la figure 3 est une vue partielle de dessous de l'extrémité de ladite pièce coopérante du dispositif de la figure 1,
- la figure 4 illustre schématiquement le mode d'utilisation du dispositif de la figure 1,
- la figure 5 représente une vue schématique en coupe d'un second mode de réalisation du dispositif, avec un réceptacle en forme de fente fine,
- la figure 6 est une vue en coupe longitudinale verticale du dispositif de la figure 5,
- et la figure 7 schématise le mode d'utilisation du dispositif de la figure 5.

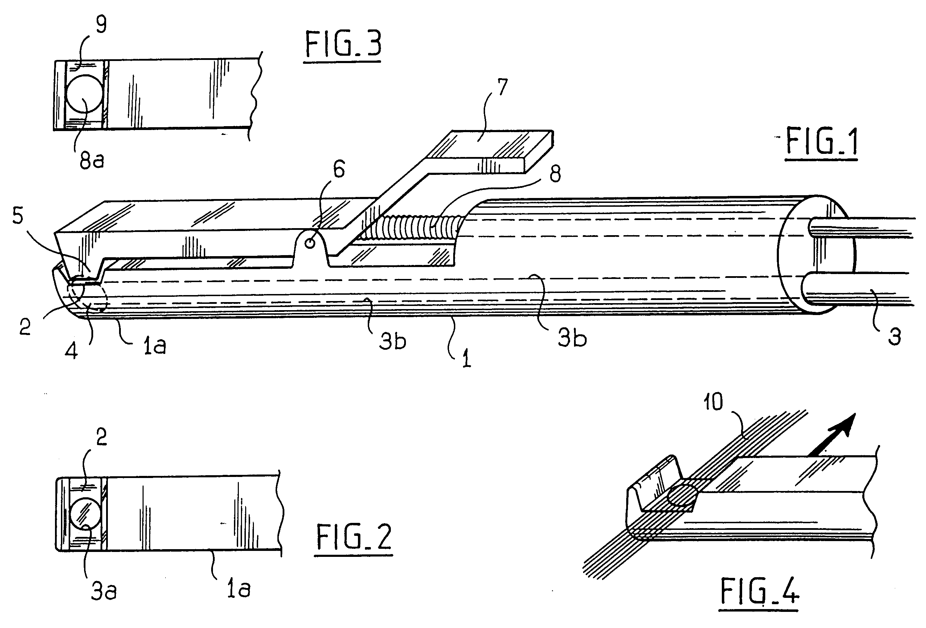

On voit sur la figure 1 que le dispositif comprend un corps allongé (1) partiellement creux dont l'extrémité antérieure (1a) de section réduite, comporte un réceptacle ayant la forme d'une rainure à fond plat (2). Un conduit (3) pour le faisceau laser débouche dans le fond de la rainure (2) après renvoi coudé à l'aide du miroir (4), et forme une lucarne de sortie (3a) à la surface de la paroi (2). Les pointillés (3b) schématisent la propagation du faisceau laser à l'intérieur du corps (1) ou peuvent représenter un guide d'onde.

Une pièce coopérante (5) mobile autour de l'axe (6) est maintenue engagée dans le réceptacle à l'aide de moyens de rappel tels qu'un ressort à tension (non représenté) et est dégageable par actionnement du levier (7) dont est munie la pièce (5) à son extrémité arrière. Un tube souple (8) est relié à une source d'air comprimé (non représenté) et débouche dans la face (9), en regard du fond de la rainure, de la pièce (5), par un orifice (8a). La partie arrière du corps (1) sert de poignée de prise en main de l'appareil par le manipulateur. Pour décolorer les cheveux, on dégage la pièce (5) pour pouvoir disposer la mèche de cheveux (10), répartie en nappe comme indiqué à la figure 4, puis on laisse la pièce (5) se réengager dans la rainure. La mèche se trouve donc disposée dans l'espace entre les faces (2) et (9), on actionne l'émetteur laser (non représenté) ainsi que le système de refroidissement et on déplace le dispositif par rapport à la mèche (ou vice-versa) pour décolorer successivement toute la mèche ou une partie dé mèche à traiter. Il est préférable d'effectuer des déplacements qui ne soient pas trop lents, par exemple de l'ordre de 0,1-5 centimètres par seconde, et d'opérer par passages successifs, ce qui permet de contrôler très facilement l'évolution de la décoloration, dans de bonnes conditions de sécurité. Mais il est également possible, avec un refroidissement efficace, d'utiliser des vitesses de déplacement plus faibles, ou des déplacements discontinus.

Le corps du dispositif peut être réalisé en tout matériau approprié, par exemple en métal léger comme l'aluminium ou en matière plastique. Le tuyau (8) est un tuyau souple classique. Il peut être relié à un système d'aspiration à la place de la source d'air comprimé.

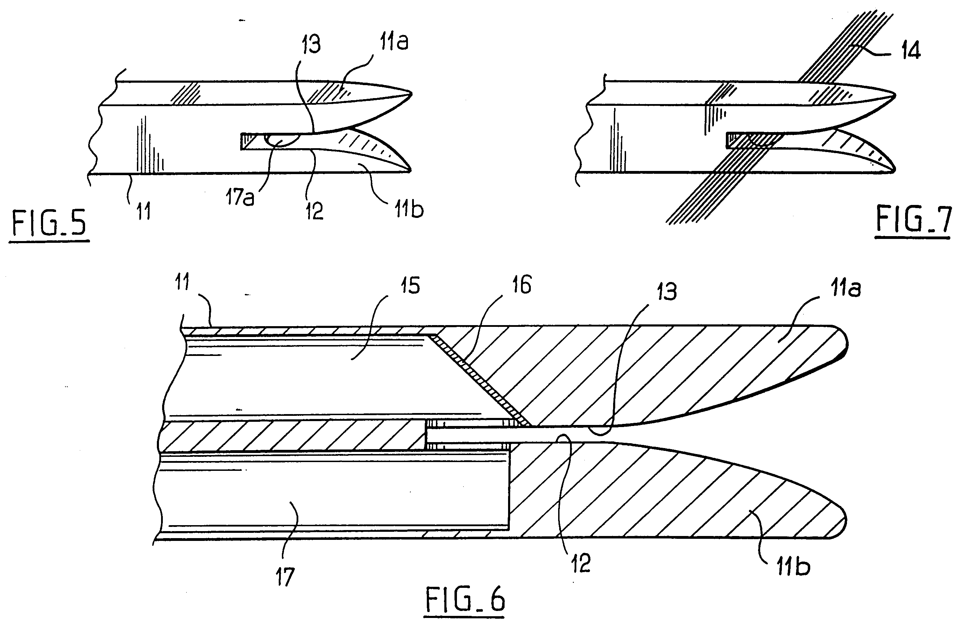

Le dispositif représenté aux figures 5 et 6 comporte un corps allongé (11) dont on n'a représenté que la partie antérieure, la partie arrière non représentée formant poignée. On voit que l'extrémité du corps (11) se sépare en deux branches (11a) et (11b) séparées par une fente dont les faces en regard (12) et (13) constituent un logement pour une mèche de cheveux (14) disposée comme indiqué à la figure 7. Comme dans le premier mode de réalisation, un canal laser (15) débouche sur la face (13) de la fente après renvoi par le miroir (16), et un canal (17) relié à une source d'air comprimé débouche à la surface de la paroi (12) en (17a).

On utilise ce dispositif de façon analogue au précédent. Les organes de commande du faisceau laser et du circuit de refroidissement, qui peuvent être couplés, peuvent être installés sur le dispositif ou dans un organe de commande séparé qui peut être éventuellement actionné au pied. On peut également prévoir des dispositif d'asservissement de la puissance laser, en fonction de la couleur de la mèche à traiter, cet asservissement pouvant être réalisé de façon automatique après lecture de la couleur par un détecteur approprié.

Les exemples suivants illustrent l'invention.

**EXEMPLE 1 :**

Dans cet exemple et dans les exemples suivants, on utilise des mèches de cheveux de 0,25 g, ayant une longueur de 20 cm.

L'appareil utilisé est un appareil du type représenté à la figure 1.

La source de rayonnement laser est un laser Surelite Continuum longueur d'onde : 532 nm ; fréquence des tirs : 1 Hz ; diamètre du faisceau : 5 mm ; durée de l'impulsion ; 4 ns.

Avec cet appareillage, on a étudié les plages de décoloration optimale, selon la couleur des cheveux à traiter.

Les gammes d'énergie par $cm^2$ pour une impulsion, sont celles qui sont utilisables pour décolorer effectivement les cheveux.

En-dessous de la valeur minimum, il n'y a pas de décoloration notable. Au-dessus de la valeur maximum, la fibre du cheveu éclate ou est fissurée (les dommages sont visibles, selon leur importance, à la loupe binoculaire, au microscope ou au microscope électronique).

Les résultats sont résumés dans le tableau (I) suivant :

TABLEAU (I)

| Cheveux | énergie/$cm^2$ pour 1 impulsion (en J/$cm^2$) |
|---|---|
| japonais | 0,2 à 0,35 |
| châtain foncé | 0,2 à 0,4 |
| châtain clair | 0,15 à 0,5 |
| blond foncé | 0,15 à 0,7 |
| blond clair | 0,1 à 1,2 |

Par ailleurs, l'absorption de l'énergie lumineuse de la mélanine varie avec la longueur d'onde : elle diminue lorsque la longueur d'onde augmente, de sorte que les cheveux supportent sans dégradation une densité d'énergie incidente plus élevée lorsque la longueur d'onde augmente. L'étude expérimentale a montré que la densité d'énergie maximale supportable par les cheveux sans éclatement de la fibre kératinique, pour un rayonnement de longueur d'onde λ, est sensiblement celle indiquée dans le tableau ci-dessus, multipliée par un facteur

$$\frac{\lambda}{532}$$

où $\lambda$ est exprimée en nanomètres. Cette loi de variation avec la longueur d'onde vaut également pour la relation eritre la densité d'énergie incidente et l'efficacité de la décoloration : la densité d'énergie capable de décolorer les cheveux d'un type donné, pour la longueur d'onde $\lambda$, est sensiblement égale à la densité d'énergie permettant d'obtenir une décoloration analogue avec un rayonnement de longueur d'onde 532 nm, multipliée par ledit facteur

$$\frac{\lambda}{532}$$

Pour des cheveux colorés artificiellement, il faut utiliser des densités d'énergie assez élevées, généralement au moins égales à 0,8 $J/cm^2$ par impulsion. Si les cheveux ont été colorés sans décoloration préalable, il faut tenir compte de leur teinte naturelle. Par exemple, si la teinte naturelle des cheveux était châtain clair, il faut d'abord utiliser une densité d'énergie non supérieure à 0,5 $J/cm^2$ (voir tableau 1 ci-dessus) pour décolorer la mélanine. Ce n'est qu'ensuite qu'on pourra utiliser une densité d'énergie supérieure (1 $J/cm^2$ ou plus) pour détruire le colorant artificiel. Si l'on appliquait dès le début cette densité d'énergie élevée, les cheveux éclateraient.

**EXEMPLE 2 :**

On opère comme à l'exemple 1, avec un laser Quanta Ray (Spectra Physics) ; longueur d'onde : 532 nm ; fréquence des tirs : 50 Hz ; diamètre du faisceau : 8 mm ; durée de l'impulsion : 7 ns.

Le refroidissement est assuré par un débit d'air de 0,5 litre par seconde.

Avec cet appareil, la puissance crête par unité de surface correspondant à une décoloration optimale pour des cheveux châtain foncé est de l'ordre de 40 $MW/cm^2$, soit une énergie par unité de surface de 0,3 $J/cm^2$.

**EXEMPLE 3 :**

On opère comme précédemment, avec un laser BMI présentant les caractéristiques suivantes : longueur d'onde : 523 nm ; fréquence des tirs : 20 Hz ; diamètre du faisceau : 3 mm ; durée de l'impulsion : 30 ps ; refroidissement par flux d'hélium.

On a obtenu une décoloration optimale de cheveux châtain foncé avec une énergie par unité de surface de 0,28 $J/cm^2$.

Si on opère en coupant le débit d'hélium, on observe une dégradation du cheveu avec fusion des écailles, visible à la loupe ou au microscope.

**EXEMPLE 4 :**

Avec l'appareillage décrit à l'exemple 1, on a décoloré des mèches de cheveux de 0,2 g, longueur 20 cm, en déplaçant lentement la pince à décolorer le long de la mèche.

Refroidissement par air; débit 0,25 litre par seconde.

Fréquence des impulsions : 10 Hz.

Avec des cheveux châtain foncé et un passage de la totalité de la mèche en 5 minutes environ, on a obtenu une décoloration complète après 5 passages, en utilisant une puissance crête correspondant à une énergie de 0,35 $J/cm^2$ par impulsion.

Pour des cheveux blond clair, on a obtenu une décoloration complète après un seul passage d'une durée d'une minute environ.

Avec des cheveux châtain foncé, on a fait des mesures de solubilité alcaline sur les cheveux naturels, les cheveux décolorés par rayonnement laser et les cheveux décolorés par voie chimique (peroxyde d'hydrogène).

La solubilité alcaline sert à caractériser l'état de dégradation des cheveux. Les mèches sont immergées pendant 30 minutes à 65°C dans une solution 0,1 N d'hydroxyde de sodium, puis rincées 3 fois par immersion pendant 5 minutes dans de l'eau distillée, et enfin séchées à l'étuve à 105°C jusqu'à poids constant. La perte de poids des cheveux, en %, représente la solubilité alcaline.

On a également procédé à une détermination de l'acide cystéique. Après un traitement d'hydrolyse des cheveux à chaud en milieu acide, on mesure la quantité d'acide cystéique passée en solution, en séparant les acides aminés sur résine échangeuse d'ions et en effectuant une réaction colorée à la ninhydrine. Sur cheveux naturels, le taux d'acide cystéique est entre 0 et 0,8 %. Sur cheveux dégradés, ce taux augmente.

On a constaté que la décoloration laser ne modifie pratiquement pas la solubilité alcaline, ni la teneur en acide cystéique alors qu'elles sont fortement augmentées après décoloration par voie chimique.

**EXEMPLE 5 :**

On prend quatre mèches de cheveux européens naturels de 0,25 g ayant une longueur de 20 cm. On fait subir à la mèche n°1 trois traitements de décoloration (Formule n°1) de 30 minutes.

| Formule n° 1 : | |
|---|---|
| 60 g de la formule suivante : | |
| - Nonylphénol oxyéthyléné (4 OE) | 25 g |
| - Nonylphénol oxyéthyléné (9 OE) | 20 g |
| - Acide laurique oxyéthyléné (2 OE) | 7 g |
| - Acide oléique | 30 g |
| - Propylène glycol | 12 g |
| - 1-hydroxy-4 phényl aminoanthraquinone | 0,05 g |
| - Ammoniaque (20 %) | 7 g |
| - Eau déminéralisée qsp | 100 g |
| mélangé à 50 g de la poudre suivante : | |
| - Persulfate de sodium | 30 g |
| - Persulfate de potassium | 25 g |
| - Silicate de sodium anhydre | 3 g |
| - Silice colloïdale | 0,4 g |
| - Chlorure d'ammonium | 12 g |
| - 1-hydroxy-4 phénylamino anthraquinone | 0,3 g |
| et mélangé en outre à 120 ml de la solution suivante | |
| - Eau oxygénée à 30 volumes* | 100 g |
| - Acide phosphorique q.s.p. | pH 3 |

*solution aqueuse de $H_2O_2$ 2,7 N

La mèche n° 2 subit une décoloration par irradiation laser, conformément à l'invention, à l'aide de l'appareil du type représenté à la figure 1. La source de rayonnement laser est un laser Surelite Continum ; longueur d'onde : 532 nm ; fréquence de tirs : 1 Hz ; diamètre du faisceau: 5 mm ; durée de l'impulsion : 4 ns.

Les deux mèches présentent des éclaircissements équivalents. (C'est pour obtenir une décoloration de la mèche n° 1 équivalente à celle de la mèche n° 2 qu'il a été nécessaire de traiter trois fois la mèche n° 1).

On enroule les deux mèches, et une troisième non décolorée sur rouleaux de 9 mm de diamètre puis on applique la première lotion de permanente (Formule n° 2) qu'on laisse poser 15 minutes. On rince, on applique la seconde lotion de permanente (Formule n° 3). On laisse poser 5 minutes puis on rince.

| Formule n° 2 : | |
|---|---|
| - Acide thioglycolique | 10 g |
| - Ammoniaque q.s. | pH 9 |

(suite)

| Formule n° 2 : | |
|---|---|
| - Eau déminéralisée q.s. | 100 g |

| Formule n° 3 : | |
|---|---|
| - Eau oxygénée q.s. | 8 volumes |
| - Acide citrique q.s. | pH 3 |
| - Eau déminéralisée q.s | 100 g |

La mèche n° 4, n'ayant subi aucun traitement, sert de témoin.
On lave les quatre mèches et on les compare.

*Frisure :*

Les mèches 2 et 3 présentent de bonnes frisures. La première mèche n'est pratiquement pas frisée. On a suspendu par une extrémité les quatre mèches humides et peignées. Après quelques minutes, les mèches 2 et 3 présentent un aspect bouclé. Les boucles sont toniques. L'extrémité inférieure des cheveux a donc remonté. La première mèche ne remonte pas. Après quelques minutes, on mesure la hauteur des mèches (distance de la racine à la pointe sans étirer la mèche).

| *Mèches :* | hauteur de la mèche sèche |
|---|---|
| 1. Décoloration chimique puis permanente | 30 cm |
| 2. Décoloration au laser puis permanente | 22 cm |
| 3. Permanente | 22 cm |
| 4. Naturelle | 31 cm |

Les mèches n° 2, 3 et 4 présentent un toucher agréable et sain. La mèche n° 1 présente un toucher anormal, glissant ou "ramolli".

**EXEMPLE 6 :**

On effectue un méchage sur une chevelure de personne européenne. Pour cela, on dégage environ 50 mèches de 0,5 g que l'on traite par le procédé de décoloration par irradiation laser conformément à l'invention.
Une fois les cheveux rincés, on enroule l'ensemble des cheveux sur bigoudis de 9 mm de diamètre et on procède à une permanente forte (lotion 2 puis lotion 3 ; voir exemple 1). On enlève les bigoudis et on rince. On constate que les cheveux sont bouclés de façon homogène. Les mèches décolorées sont tout aussi bouclées que les mèches naturelles. Le toucher de l'ensemble des cheveux est bon.

**EXEMPLE 7 :**

On effectue sur une chevelure de personne européenne un méchage sur de nombreuses petites mèches, appelé "balayage". Pour cela, on dégage, à l'aide d'un peigne, environ 150 mèches de 0,15 g que l'on traite par le procédé de décoloration par irradiation laser conformément à l'invention.
Une fois les cheveux rincés, on sèche les cheveux.
Deux semaines après, on enroule l'ensemble des cheveux sur bigoudis de 9 mm de diamètre et on procède à une permanente forte (lotion 2 puis lotion 3 de l'exemple 1). On enlève les bigoudis et on rince. On constate que les cheveux sont bouclés de façon homogène. Les mèches décolorées sont tout aussi bouclées que les mèches naturelles. Le toucher de l'ensemble des cheveux est bon.

**EXEMPLE 8 :**

On prend deux mèches de cheveux européens naturels châtain et une mèche de cheveux européens naturels 70 % de gris. On fait subir à la première trois traitements de décoloration (formule n°1) de 30 minutes mettant en oeuvre de l'eau oxygénée, des persels et de l'ammoniaque.

La seconde mèche subit une décoloration par irradiation laser, conformément à l'invention.

Les deux premières mèches présentent des éclaircissements équivalents.

On teint les trois mèches avec une coloration mettant en oeuvre un mélange poids pour poids de formules n°4 et 5 :

| Formule n°4 | |
|---|---|
| - Paraphénylène diamine | 0,1 g |
| - Métahydroxyphénol | 0,3 g |
| - Métahydroxyphénol | 0,1 g |
| - Métaaminophénol | 0,1 g |
| - 2-méthyl 5-($\beta$-hydroxyéthyl) amino phénol | 0,2 g |
| - Ammoniaque (20 %) | 10 g |
| - Alcool éthylique | 10 g |
| - Eau déminéralisée q.s. | 100 g |

| Formule n°5 | |
|---|---|
| - Eau oxygénée à 20 volumes | 100 g |
| - Acide phosphorique q.s. | pH 2,5 |

On observe que la première mèche est très fortement teinte. La nuance est un cuivré-doré. Les deux suivantes sont moyennement teintes et bien que la mèche traitée par l'irradiation laser puis par la teinture soit un peu plus foncée que la mèche naturelle traitée par la teinture, les nuances sont très proches (châtain-cuivré).

De plus, lorsqu'on shampooine (6 shampooings) les trois mèches, on constate que les teintes des mèches 2 et 3 ont une très bonne tenue. A l'inverse, la nuance de la première mèche se délave pour donner une teinte jaune.

On rappelle qu'une eau oxygénée dite "à 20 volumes" est une solution aqueuse de peroxyde d'hydrogène 1,8 N.

**EXEMPLE 9 :**

On prend deux mèches de cheveux européens naturels châtain et une mèche de cheveux européens naturels 70 % de gris. On fait subir à la première, trois traitements de décoloration (formule n°1) de 30 minutes en mettant en oeuvre de l'eau oxygénée, des persels et de l'ammoniaque.

La seconde mèche subit une décoloration par irradiation laser, conformément à l'invention.

Les deux premières mèches présentent des éclaircissements équivalents.

Les trois mèches sont mises sous rouleaux de mise en plis.

On traite les trois mèches avec une laque mettant en oeuvre un copolymère anionique (formule n°6).

| Formule n°6 | |
|---|---|
| - Copolymère méthylvinyléther/maléate de butyle vendu sous la dénomination "Gantrez ES425 L®" (I.S.P.), neutralisé à 100 % par de l'aminométhyl propanol | 5 g |
| - Alcool éthylique q.s. | 100 g |

la formule étant mise sous pression de diméthyléther (30 g pour 100 g de formule).

On constate que les mèches 2 et 3 sont plus douces et plus faciles à démêler que la première.

**EXEMPLE 10 :**

On prend deux mèches de cheveux européens naturels châtain et une mèche de cheveux européens naturels 70 % de gris. On fait subir à la première, trois traitements de décoloration (formule n°1) de 30 minutes mettant en oeuvre de l'eau oxygénée, des persels et de l'ammoniaque.

La seconde mèche subit une décoloration par irradiation laser, conformément à l'invention.

Les deux premières mèches présentent des éclaircissements équivalents.

Les trois mèches subissent un traitement de 8 shampooings (formule n°7) (0,5 g de shampooing par gramme de cheveux).

| Formule n°7 | |
|---|---|
| - Lauryl éther sulfate de sodium polyoxyéthyléné (2,2 OE) en solution aqueuse à 70% de matière active | 15 g (MA) |
| - Laurylbétaïne | 2,5 g |
| - Eau déminéralisée q.s. | 100 g |

On constate que les mèches 2 et 3 sont plus douces et plus faciles à démêler que la première.

OE signifie : oxyde d'éthylène
MA signifie : matière active

**Revendications**

1. Procédé de traitement des cheveux comprenant une décoloration desdits cheveux pour obtenir des cheveux décolorés, et l'application d'un traitement cosmétique, autre qu'une décoloration, sur lesdits cheveux décolores, caractérisé par le fait que, dans le but d'améliorer les résultats dudit traitement cosmétique, ladite décoloration consiste essentiellement à irradier lesdits cheveux à l'aide d'un rayonnement laser.

2. Procédé selon la revendication précédente, caractérisé par le fait que ledit traitement cosmétique est choisi parmi l'un au moins des traitements suivants opération de déformation permanente ou de teinture, shampooing ou application d'une composition de conditionnement, d'une lotion de mise en plis ou d'une laque.

3. Procédé selon lit revendications 1 ou 2, dans lequel les cheveux ont été décolorés au moins partiellement par irradiation d'une mèche ou partie de mèche à l'aide d'un faisceau laser de puissance suffisante pour décolorer les cheveux.

4. Procédé selon la revendication 3, dans lequel, pour effectuer la décoloration :

   - on traite une zone de ladite partie de mèche par irradiation à l'aide d'un faisceau laser émis sous forme d'impulsions pour décolorer au moins partiellement les cheveux de ladite zone, par dégradation de la mélanine des cheveux,

- le cas échéant, par déplacement relatif de ladite mèche par rapport audit faisceau laser, on traite successivement, de façon analogue, une ou plusieurs autres zones de façon à traiter la totalité de ladite partie de mèche,

- on répète éventuellement les traitements précédents jusqu'à obtention du degré de décoloration souhaité pour ladite mèche ou partie de mèche,

- on opère avec un rayonnement laser de puissance suffisante pour fournir, par impulsion, une densité d'énergie de 0,1 à 1,2 J/cm$^2$ à 532 nm, ladite densité d'énergie choisie n'étant pas supérieure à un seuil au-delà duquel la fibre kératinique des cheveux est endommagée, ledit seuil étant d'autant plus faible que la couleur naturelle des cheveux à traiter est plus foncée, étant entendu que les valeurs de densité d'énergie sont données ici pour un rayonnement de longueur d'onde de 532 nm et doivent être multipliées par un facteur de correction égal à :

$$\frac{\lambda}{532}$$

lorsque le rayonnement utilisé a une longueur d'onde $\lambda$ (exprimée en nanomètres) autre que 532 nm.

5. Procédé selon la revendication 4, caractérisé par le fait que l'on opère de façon à ne pas dépasser une densité d'énergie maximum par impulsion qui est de l'ordre de :

- 0,35 J/cm$^2$ pour les cheveux de type japonais,
- 0,4 J/cm$^2$ pour les cheveux châtain foncé,
- 0,5 J/cm$^2$ pour les cheveux châtain clair,
- 0,7 J/cm$^2$ pour les cheveux blond foncé,
- 1,2 J/cm$^2$ pour les cheveux blond clair,

les valeurs de densité d'énergie étant données ici pour un rayonnement de longueur d'onde de 532 nm.

6. Procédé selon la revendication précédente, caractérisé par le fait que l'on opère de façon à obtenir une densité d'énergie (en joules/cm$^2$), par impulsion, de :

- 0,2-0,35 pour les cheveux de type japonais,
- 0,2-0,4 pour les cheveux châtain foncé,
- 0,15-0,5 pour les cheveux châtain clair,
- 0,15-0,7 pour les cheveux blond foncé,
- 0,1-1,2 pour les cheveux blond clair,

les valeurs de densité d'énergie étant données ici pour un rayonnement de longueur d'onde de 532 nm.

7. Procédé selon l'une quelconque des revendications précédentes, caractérisé par le fait qu'en outre, lors de l'étape de décoloration, lorsque les cheveux a décolorer sont teints à l'aide d'un agent colorant, on effectue une étape supplémentaire d'irradiation des cheveux, de façon analogue à celle décrite dans l'une quelconque des revendications précédentes, mais avec une densité d'énergie plus élevée, suffisante pour détruire ou dégrader l'agent colorant.

8. Procédé selon la revendication 7, caractérisé par le fait que ladite densité d'énergie plus élevée est au moins égale à 0,8 J/cm$^2$ par impulsion à 532 nm.

9. Procédé selon l'une quelconque des revendications 3 à 8, caractérisé par le fait que, lors de la décoloration, pour éviter un échauffement local dommageable pour les cheveux, on limite la fréquence des impulsions et/ou on procède à des déplacements relatifs suffisants de la mèche à décolorer par rapport au faisceau laser, et/ou on refroidit les cheveux dans la zone irradiée.

10. Procédé selon l'une quelconque des revendications 3 à 9, caractérisé par le fait que, lors de l'étape de décoloration, on dispose préalablement ladite mèche de façon à former une nappe de cheveux, et que l'on décolore les cheveux en irradiant au moins une des faces de ladite nappe.

11. Procédé selon l'une quelconque des revendications 3 à 10, caractérisé par le fait que, lors de l'étape de décoloration :

- on opère à l'aide d'un dispositif de traitement comprenant un réceptacle pour la mèche de cheveux à décolorer; la surface dudit réceptacle comportant un orifice pour la sortie dudit faisceau laser,

- on applique, sur ai moins une partie de leur longueur, les cheveux de ladite mèche étalés en nappe sur la surface dudit réceptacle, de façon qu'une zone à décolorer soit en regard dudit orifice,
- on procède à l'irradiation de ladite zone,
- et par déplacements relatifs de ladite mèche par rapport au faisceau laser, on procède successivement à l'irradiation des autres zones à décolorer.

12. Procédé selon la revendication 11, caractérisé par le fait que lesdits déplacements relatifs comprennent des déplacements relatifs de ladite mèche par rapport audit réceptacle.

13. Procédé selon l'une quelconque des revendications 9 et 10, caractérisé par le fait que lesdits déplacements relatifs sont obtenus en faisant varier la direction du faisceau laser de façon à effectuer des balayages de la zone à décolorer.

14. Procédé selon l'une quelconque des revendications 9 à 13, caractérisé par le fait que l'on refroidit les cheveux par circulation d'un fluide.

15. Procédé selon la revendication 14, caractérisé par le fait que l'on refroidit les cheveux par création d'un flux gazeux dans la zone irradiée.

16. Utilisation de la décoloration des cheveux à l'aide d'un rayonnement laser pour améliorer les résultats des traitements cosmétiques, autres qu'une décoloration, effectués sur cheveux décolorés.

17. Utilisation selon la revendication précédente, caractérisée par le fait que ladite décoloration est effectuée selon le procédé de l' une quelconque des revendications 3 à 15.

## Claims

1. Process for the treatment of hair, which comprises bleaching the said hair in order to obtain bleached hair, and the application of a cosmetic treatment, other than bleaching, on the said bleached hair, characterized in that, for the purpose of improving the results of the said cosmetic treatment, the said bleaching consists essentially in irradiating the said hair using laser radiation.

2. Process according to the preceding claim, characterized in that the said cosmetic treatment is chosen from at least one of the following treatments: permanent-reshaping operation or dyeing operation, shampooing or application of a conditioning composition, a setting lotion or a lacquer.

3. Process according to Claims 1 or 2, in which the hair has been bleached, at least partially, by irradiation of a lock or portion of lock using a laser beam of sufficient power to bleach the hair.

4. Process according to Claim 3, in which, in order to perform the bleaching:

- an area of the said portion of lock is treated by irradiation using a laser beam emitted in the form of pulses in order to bleach, at least partially, the hair in the said area, by degradation of the melanin in the hair,
- if required, by relative movement of the said lock with respect to the said laser beam, one or more other areas are treated in succession, in a similar way, so as to treat the totality of the said portion of lock,
- the above treatments are possibly repeated until the desired degree of bleaching is obtained for the said lock or portion of lock,
- the operation is performed with laser radiation of sufficient power to deliver, per pulse, an energy density of 0.1 to 1.2 $J/cm^2$ at 532 nm, the said chosen energy density not being greater than a threshold above which the keratinous fibre of the hair is damaged, the said threshold being lower the darker the natural colour of the hair to be treated, it being understood that the energy density values are given here for radiation of 532 nm wavelength and have to be multiplied by a correction factor equal to:

$$\frac{\lambda}{532}$$

when the radiation used has a wavelength $\lambda$ (expressed in nanometres) other than 532 nm.

5. Process according to Claim 4, characterized in that the operation is performed so as not to exceed a maximum energy density per pulse which is of the order of:

- 0.35 J/cm$^2$ for hair of the Japanese type,
- 0.4 J/cm$^2$ for dark chestnut hair,
- 0.5 J/cm$^2$ for light chestnut hair,
- 0.7 J/cm$^2$ for dark blond hair,
- 1.2 J/cm$^2$ for light blond hair,

the energy density values being given here for radiation of 532 nm wavelength.

6. Process according to the preceding claim, characterized in that the operation is performed so as to obtain an energy density (in joules/cm$^2$), per pulse, of:

- 0.2 - 0.35 for Japanese-type hair,
- 0.2 - 0.4 for dark chestnut hair,
- 0.15 - 0.5 for light chestnut hair,
- 0.15 - 0.7 for dark blond hair,
- 0.1 - 1.2 for light blond-hair,

the energy density values being given here for radiation of 532 nm wavelength.

7. Process according to any one of the preceding claims, characterized in that, furthermore, during the bleaching step, when the hair to be bleached is dyed using a colouring agent, an additional step of irradiation of the hair is performed, in a way similar to that described in any one of the preceding claims, but with a higher energy density, sufficient to destroy or degrade the colouring agent.

8. Process according to Claim 7, characterized in that the said higher energy density is at least equal to 0.8 J/cm$^2$ per pulse at 532 nm.

9. Process according to any one of Claims 3 to 8, characterized in that, during bleaching, in order to prevent local heating which can damage the hair, the frequency of the pulses is limited and/or sufficient relative movements of the lock to be bleached are carried out with respect to the laser beam, and/or the hair is cooled in the irradiated area.

10. Process according to any one of Claims 3 to 9, characterized in that, during the bleaching step, the said lock is arranged beforehand so as to form a ribbon of hair and in that the hair is bleached by irradiating at least one of the faces of the said ribbon.

11. Process according to any one of Claims 3 to 10, characterized in that, during the bleaching step:

- the operation is performed using a treatment device which includes a receptacle for the lock of hair to be bleached, the surface of the said receptacle having an orifice for the exit of the said laser beam,
- the hair of the said lock, spread out in the form of a ribbon, is applied, over at least part of its length, to the surface of the said receptacle so that an area to be bleached is opposite the said orifice,
- irradiation of the said area is then carried out,
- and, by relative movements of the said lock with respect to the laser beam, irradiation of the other areas to be bleached is carried out in succession.

12. Process according to Claim 11, characterized in that the said relative movements include relative movements of the said lock with respect to the said receptacle.

13. Process according to either of Claims 9 and 10, characterized in that the said relative movements are obtained by varying the direction of the laser beam so as to perform scanning of the area to be bleached.

14. Process according to any one of Claims 9 to 13, characterized in that the hair is cooled by circulation of a fluid.

15. Process according to Claim 14, characterized in that the hair is cooled by creating a gas flow in the irradiated area.

**16.** Use of the bleaching of hair using laser radiation in order to improve the results of cosmetic treatments, other than bleaching, performed on bleached hair.

**17.** Use according to the preceding claim, characterized in that the said bleaching is performed according to the process of any one of Claims 3 to 15.

## Patentansprüche

**1.** Verfahren zu Behandlung von Haaren, umfassend eine Entfärbung der Haare zum Erhalt von entfärbten Haaren und die Anwendung einer anderen kosmetischen Behandlung als der Entfärbung auf den entfärbten Haaren, dadurch gekennzeichnet, daß zum Zweck der Verbesserung der Resultate der kosmetischen Behandlung die Entfärbung im wesentlichen aus der Bestrahlung mit einem Laserstrahl besteht.

**2.** Verfahren gemäß dem vorstehenden Anspruch, dadurch gekennzeichnet, daß die kosmetische Behandlung unter mindestens einer der folgenden Behandlungen gewählt ist: Verfahren zum permanenten Verformen oder Färben, dem Schamponieren oder der Anwendung einer Konditionerzusammensetzung, einer Welllösung oder eines Lacks.

**3.** Verfahren gemäß den Ansprüchen 1 oder 2, bei dem die Haare mindestens teilweise durch Bestrahlung einer Locke oder einer Lockenpartie mit Hilfe eines Laserbündels mit zum Entfärben der Haare ausreichender Stärke entfärbt werden.

**4.** Verfahren gemäß Anspruch 3, bei dem man zum Bewirken der Entfärbung:

- eine Zone der Lockenpartie durch Bestrahlung mit Hilfe eines Laserbündels behandelt, das in Form von Pulsen emittiert wird, zum mindestens teilweisen Entfärben der Haare der Zone, und zwar durch Abbau des Melanins der Haare,
- falls erforderlich, durch relative Verlagerung der Locke bezogen auf das Laserbündel sukzessive auf analoge Weise eine oder mehrere weitere Zonen auf diese Art und Weise behandelt, wenn man die Gesamtheit der Lockenpartie behandelt,
- gegebenenfalls die vorstehenden Behandlungen wiederholt, bis ein für die Locke oder Lockenpartie geeigneter Entfärbungsgrad erzielt ist,
- mit einem Laserstrahl arbeitet, der ausreichend Kraft pro Impuls zum Bilden einer Energiedichte von 0,1 bis 1,2 J/cm$^2$ bei 532 nm aufweist, wobei die Energiedichte so gewählt ist, daß sie eine Schwelle nicht übersteigt, oberhalb derer die Keratinfaser der Haare beschädigt wird, wobei die Schwelle um so schwächer ist, je dunkler die natürliche Farbe der behandelten Haare ist,
- wobei außerdem die Werte der Energiedichte hier für eine Bestrahlung der Wellenlänge von 523 nm gegeben sind, und mit einem Korrekturfaktor entsprechend:

$$\lambda/532$$

multipliziert werden müssen, wenn die verwendetete Strahlung eine andere Wellenlänge $\lambda$ (ausgedrückt in nm) als 532 nm hat.

**5.** Verfahren gemäß Anspruch 4, dadurch gekennzeichnet, daß man in der Art und Weise vorgeht, daß maximale Energiedichten pro Impuls nicht überschritten werden, die in der Größenordnung liegen:

- 0,35 J/cm$^2$ für Haare vom japanischen Typ,
- 0,4 J/cm$^2$ für dunkel-dunkelblonde Haare,
- 0,5 J/cm$^2$ für heller dunkelblonde Haare,
- 0,7 J/cm$^2$ für dunkelblonde Haare, und
- 1,2 J/cm$^2$ für hellblonde Haare,

wobei die Werte der Energiedichten hier für eine Bestrahlung der Wellenlänge von 532 nm gegeben sind.

**6.** Verfahren gemäß dem vorstehenden Anspruch, dadurch gekennzeichnet, daß man so vorgeht, daß eine Energiedichte (in J/cm$^2$) von:

- 0,2 bis 0,35 für Haare vom japanischen Typ,
- 0,2 bis 0,4 für dunkel-dunkelblonde Haare,
- 0,15 bis 0,5 für heller dunkelblonde Haare,
- 0,15 bis 0,7 für dunkelblonde Haare, und
- 0,1 bis 1,2 für hellblonde Haare,

pro Impuls erhalten wird, wobei die Werte der Energiedichte hier für eine Bestrahlung mit der Wellenlänge von 532 nm gegeben sind.

7. Verfahren gemäß einem der vorstehenden Ansprüche, dadurch gekennzeichnet, daß man zusätzlich während des Schrittes der Entfärbung, wenn die entfärbten Haare mit Hilfe eines Färbungsmittels getönt sind, einen Schritt der unterstützenden Bestrahlung der Haare auf analoge Weise wie in einem der vorstehenden Ansprüche beschrieben bewirkt, aber mit einer höheren Energiedichte, die ausreicht zum Zerstören oder Abbauen des Färbungsmittels.

8. Verfahren gemäß Anspruch 7, dadurch gekennzeichnet, daß die erhöhte Energiedichte mindestens 0,8 J/cm$^2$ pro Impuls bei 532 nm entspricht.

9. Verfahren gemäß einem der Ansprüche 3 bis 8, dadurch gekennzeichnet, daß man bei der Bestrahlung, um eine für die Haare schädliche lokale Erwärmung zu vermeiden, die Frequenz der Impulse einschränkt und/oder man mit ausreichenden relativen Verlagerungen der zu entfärbenden Locke bezogen auf das Laserbündel vorgeht und/oder man die Haare in der bestrahlten Zone kühlt.

10. Verfahren gemäß einem der Ansprüche 3 bis 9, dadurch gekennzeichnet, daß man beim Schritt der Entfärbung vorzugsweise die Locke in der Art und Weise anordnet, daß sie eine glatte Fläche aus Haaren bildet und man die Haare entfärbt, indem man mindestens eine Seite dieser Fläche bestrahlt.

11. Verfahren gemäß einem der Ansprüche 3 bis 10, dadurch gekennzeichnet, daß man beim Schritt des Entfärbens:

- mit Hilfe einer Vorrichtung zur Behandlung arbeitet, die eine Aufnahmestelle für die zu entfärbende Haarlocke aufweist, wobei die Oberfläche dieser Aufnahmestelle eine Öffnung für den Ausgang des Laserbündels aufweist,
- man mindestens auf einen Teil ihrer Länge die Haare der Locke auf der Oberfläche der Aufnahmestelle in der Weise ausbreitet, daß eine zu entfärbende Zone gegenüber der Öffnung liegt,
- man mit der Bestrahlung der Zone fortfährt und
- man durch relative Verlagerung der Locke bezogen auf das Laserbündel sukzessive mit der Bestrahlung der anderen Zonen beim Entfärben fortfährt.

12. Verfahren gemäß Anspruch 11, dadurch gekennzeichnet, daß die relativen Verlagerungen relative Verlagerungen der Locke bezogen auf die Aufnahmestelle umfassen.

13. Verfahren gemäß einem der Ansprüche 9 und 10, dadurch gekennzeichnet, daß die relativen Verlagerungen beim Bestrahlen durch Ändern der Richtung des Laserbündels in der Weise erhalten wird, daß man ein Überstreichen der Zone beim Entfärben bewirkt.

14. Verfahren gemäß einem der Ansprüche 9 bis 13, dadurch gekennzeichnet, daß man die Haare durch Zirkulation eines Fluids kühlt.

15. Verfahren gemäß Anspruch 14, dadurch gekennzeichnet, daß man die Haare durch Erzeugen eines Gasstroms in der bestrahlten Zone kühlt.

16. Verwendung der Entfärbung von Haaren mit Hilfe eines Laserstrahls zur Verbesserung der Resultate von anderen kosmetischen Behandlungen als der Entfärbung, die auf den entfärbten Haaren angewandt werden.

17. Verwendung gemäß dem vorstehenden Anspruch, dadurch gekennzeichnet, daß die Entfärbung gemäß einem der Ansrüche 3 bis 15 bewirkt wird.

FIG.3

9

8a

FIG.1

7

6

8

5

2

4   1a

3b   1   3b   3

FIG.2

2

3a   1a

10

FIG.4

EP 0 682 937 B1

FIG.5

FIG.7

FIG.6

EP 0 682 937 B1